# EUROPEAN PATENT SPECIFICATION

(11) **EP 1 647 242 B1**
(45) Date of publication and mention of the grant of the patent: **28.05.2008**
(21) Application number: 05256308.7
(22) Date of filing: 11.10.2005
(51) Int. Cl.: A61F 2/34

(54) **Prosthetic acetabular cups**
Prothetische Hüftpfannen
Cupules acétabulaires prothétiques

(30) Priority: 12.10.2004 GB 0422666
(43) Date of publication of application: 19.04.2006
(73) Proprietor: BENOIST GIRARD SAS, 14201 Hérouville-Saint-Clair Cédex (FR)
(72) Inventor: Raugel, Patrick, 37530 Chargé (FR); Jones, Eric, County Limerick (IE); Rushton, Neil, Cambridge (GB); Field Richard Eddy, Surrey, KT20 7UB (GB)
(74) Representative: Bridge-Butler, Alan James

(56) References cited:
- EP-A- 0 212 087
- EP-A- 0 727 195
- EP-A- 0 867 159
- EP-A- 1 025 815
- EP-A- 1 205 160
- EP-A- 1 208 819
- DE-A1- 4 227 002
- US-A- 5 658 348

## Description

This invention relates to prosthetic acetabular cups and to methods of making them.

The invention is intended to solve the long term attachment to the bone of an implant that incorporates benefits of composite materials for structural and bearing functions.

Prosthetic metallic acetabular cup implants and assemblies are usually much stiffer than the surrounding bone and this stiffness of the acetabular cup causes changes of density in the cup surrounding the bony structure. EP 0 552 949 shows an elastic acetabular cup which has now demonstrated in vivo its efficiency.

The Applicants have developed a composite material made of polyetheretherketone (PEEK) resin and 20 to 40 % of short carbon fibres, preferably with 30 % short carbon fibres. This material has demonstrated good wear resistance properties and a prosthetic bearing component comprising these materials is described in EP 1 205 160 A.

Hydroxyapatite (HAP) coating activates bone cells attachment but HAP resorbs and bone cells come directly in contact with the material of the implant (which is usually made of a titanium alloy or of the composite material described in EP 1 205 160. Some material is more prone to encourage bone cell adherence and development. Beneath the hydroxyapatite layer, the surface roughness, porosity and purity do have an effect on the bone cells development. Pure titanium with a roughness in a range of 4 to 7 µm Ra, 30 to 40 µm Rz, and 35 to 65 µm Rt is known for encouraging bone cells adherence and growth so creating a microlock between newly formed bone and the implant.

Open pores at the surface of the implant are rooms for bone trabeculae formation and deep interdigitation. This mechanical interlocking is able to provide long term attachment after complete dissolution of the hydroxyapatite coating.

Composite/plastic materials are not X-ray luscent.

DE 42 27 002 A1 shows a hip joint socket which can be made of a plastics material, for example polyethylene, and a porous metal casing element which is arranged on the convex surface of the plastic socket part and in which the plastic socket and the casing element are joined securely to one another. It goes on to state that the porous casing element may consist of a plurality of particles and is embodied as a shaped disc, plate or the like. It also states that the plate or the like can be sintered. The plastics material referred to, however, will not provide the advantages of the use of a PEEK resin and 20 - 40 % short carbon fibres which, as referred to above, have particular properties. There is no suggestion anywhere in this public document of the use of a bioactive material coating.

The construction does not therefore meet requirements of the present invention which is to provide a construction which can use PEEK resin and a bioactive coating successfully.

EP 1208 819 shows a cup construction which has a backing layer made of pure PEEK which can be sprayed with a hydroxyapatite (HA) coating but the bearing surface layer is not made from a composite material including PEEK resin and at least 20 - 40 % short carbon fibres, moreover, the PEEK backing does not have a rough or porous, outer surface.

EP-A-0 212 087 shows a hip joint socket for cement free anchoring in a pelvic bone and includes a plastic body which has a multi-layer grid which is secured to the outer surface of the plastic body for contacting a pelvic bone. One layer of the metal grid is pressed into the plastic surface for anchoring purposes and the grid may be formed of layers of wire mesh. Wire mesh is a difficult material to handle and there are problems when applying a flat sheet of mesh the plastic body having a spherical outer surface. This specification does not relate to the use of the composite material PEEK resin and 20 - 40 % short carbon fibres and difficulties associated with such materials which are intended to be overcome by the present invention.

According to the present invention a method of forming a prosthetic actebular cup includes moulding a bearing liner from a plastics material to form an inner bearing surface and attaching thereto a metallic backing layer with a roughened or porous outer surface characterised in that said plastics material is PEEK resin and 20% to 40% carbon fibres, said metallic backing layer is formed by plasma vapour deposition, sputtering metal particles on the outer non-bearing surface of the bearing layer or forming said metallic backing layer by laser sintering to provide a porous inner surface layer, a dense barrier layer and an outer layer with controlled inter-connected porosity intended for bone ingrowth, and coating the outer surface of said cup with bioactive materials by sputtering or chemical deposition.

Depending on the coating properties one or more of the following three functions is fulfilled:
- create a barrier between the composite materials and the bone cells;
- provide an appropriate roughness for bone cell attachment;
- provide open porosity for bone cells ingrowth.

All of these can be achieved with a cup made by the method as set out above.

The backing layer can be made from titanium, tantalum or niobium.

The benefit of the construction is that bioactive material encourages the bone cells apposition and development; rough surface and/or porous surface provides structure for mechanical fixation after dissolution of the bioactive layer; composite material provides elasticity for natural load distribution to the bone, and also provides highly wear resistant bearing surface; and the benefits of the metallic material is to provide an opaque marker for X-rays and a proven biological interface for good bone ongrowth/ingrowth.

The bioactive material can be hydroxyapatite (HAP) or bone morphogenic proteins (BMP).

The backing layer can be arranged to have a porosity which varies from its inner to its outer sides to form an outer porous surface.

The metallic backing layer can be formed by sputtering metal particles, they can be varied in size to form an interconnected porosity which increases towards the outer surface as the layer is built up.

The invention can be performed in many ways and some embodiments will now be described by way of example and with reference to the accompanying drawings in which :
Figure 1 is a diagrammatic cross-section through an acetabular cup according to the present invention;
Figure 2 shows an alternative method of making a cup according to the invention;
Figure 3 shows a third alternative construction and method of making it;
Figure 4 shows a fourth alternative construction and a method of making it;
Figure 5 shows a fifth alternative construction and a method of making it;
Figure 6 shows a sixth alternative construction and method of making it; and,
Figure 7 shows a seventh alternative construction and method of making it.

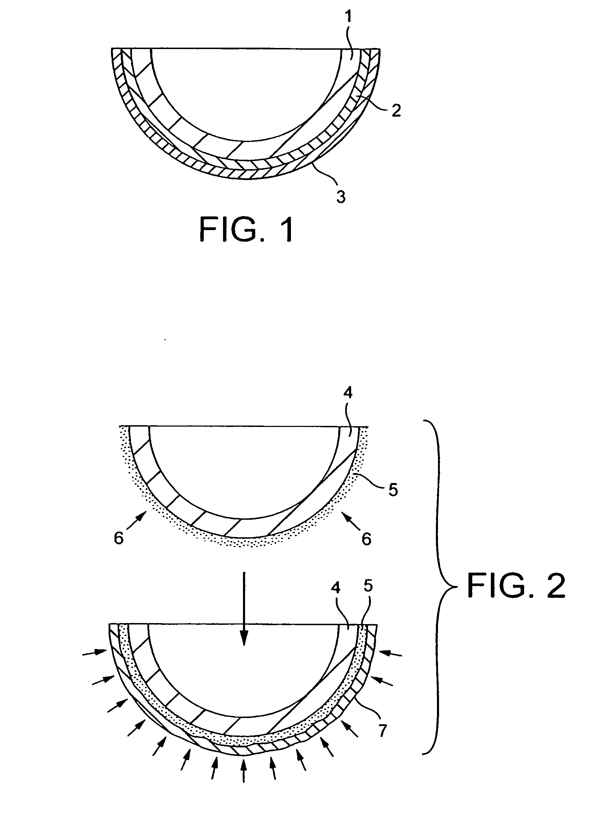

As shown in Figure 1 a prosthetic acetabular cup, according to the invention, comprises a bearing surface layer 1 which is injection moulded of a composite material including PEEK resin and at least 20 to 40 % short carbon fibres. The material can be substantially as set out in EP 1205 160, see for example pitch based carbon fibres mixed with a PEEK resin (IC Grade 150 g). The material can be pelletized and the carbon fibres can be chopped fibres with an average diameter of 8 µm and an average length of 20 µm. The pellets can be moulded into an acetabular cup and the fibre loading in the specimens can be arranged to range from 20% to 40 %. Again, if desired, the cup can be shaped as set out in EP 0 552 949 or EP 1 205 160.

Commercially pure titanium particles are then sputtered with a plasma torch under vacuum or under gas such as argon to form a backing layer 2. The outer side of the cup can be roughened prior to this metal coating. Hydroxyapatite (HAP) is then sputtered with a plasma torch onto the outer surface of the backing layer, as indicated by reference numeral 3.

The ensuing structure provides a prosthetic acetabular cup which has an inner bearing layer made from the composite material which has a natural elasticity for natural load distribution to the bone and provides a high wear-resistant bearing surface. The backing layer 2 creates a barrier between the composite material and the bone cells and provides an appropriately roughness for bone cell attachment or provides open porosity for bone cell ingrowth and the bioactive material 3 encourages the bone cells apposition and development.

The use of a metallic material for the backing layer 2 provides an opaque marker for X-rays.

Figure 2 shows a second method and construction. In this arrangement the bearing surface layer 4 is made in a similar manner to that described with regard to Figure 1, that is the composite structure is injection moulded. A second layer is then formed by sputtering commercially pure titanium particles with a plasma torch under vacuum to provide a backing layer 5, the sputtering being indicated by arrows 6. At the beginning of the coating process the size of the titanium particles is small and increases in order to form an interconnected porosity which increases over the width of the structure. The porous structure 5 is then coating with HAP by deposition in order to ensure a continuous HAP layer indicated by reference numeral 7. (Pore size : 400 µm nominal, irregular structure.)

The porosity of the layer 5 assists in providing a structure for mechanical fixation after dissolution of the bioactive layer 7.

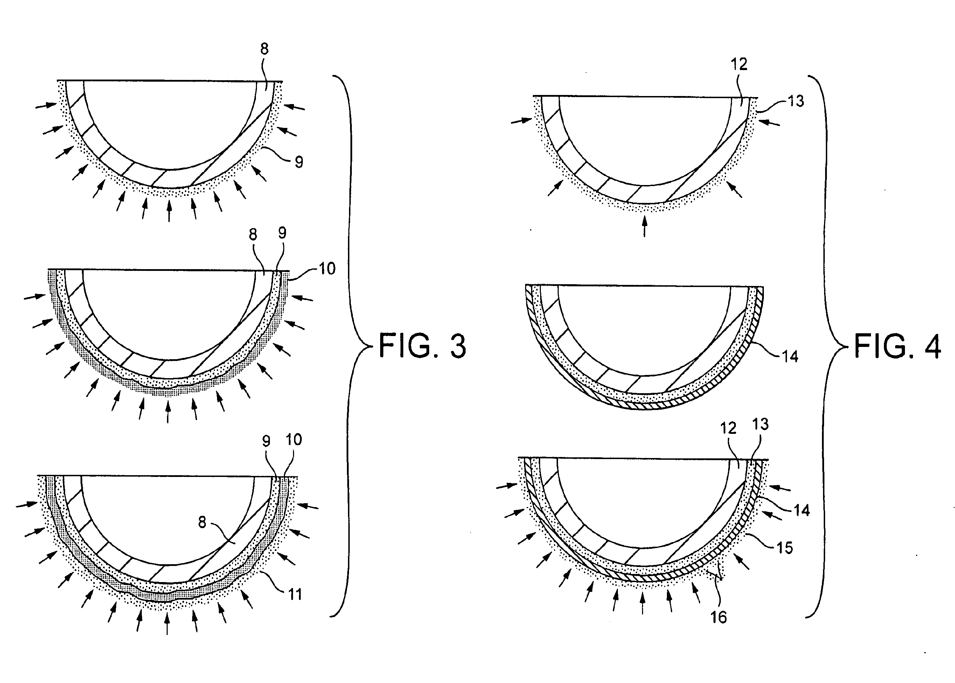

Figure 3 shows a three stage method in which the inner bearing layer 8 and backing layer 9 are made in a similar manner to that described in the method shown in Figure 2. A further layer 10 is then formed on the backing layer by sputtering a mixture of titanium powder and hydroxyapatite particles and finally the second layer 10 is then sputtered with pure hydroxyapatite powder, indicated by reference numeral 11.

The hydroxyapatite particles 11 embedded in the titanium layer 10 dissolve and are replaced by bone trabeculae that enhance the mechanical fixation of the implant.

In the description and method shown in Figure 4 the bearing layer 12 and layer 13 are made in a similar manner described with regard to Figures 1 to 3. The size of the pure titanium particles 13 and the surface roughness is smooth enough to tolerate the formation of a titanium structure 14 which is obtained by a laser sintering process, for example using the process described in US Patent Application No. US/2004/0191106. The resulting porous structure is then coated with HAP by deposition/sputtering in order to ensure a continuous HAP layer 15. The construction creates a modulus gradient from composite to HA and this can provide a better mechanical construction.

A benefit of this construction and method is that it provides a predetermined type of porosity (size, extent), some fixation fixtures can be deposited, as indicated by reference numeral 16, and a porosity, density or a combination can be provided, for example fins or spikes.

If barbs are included they can provide additional multidirectional torsional stability.

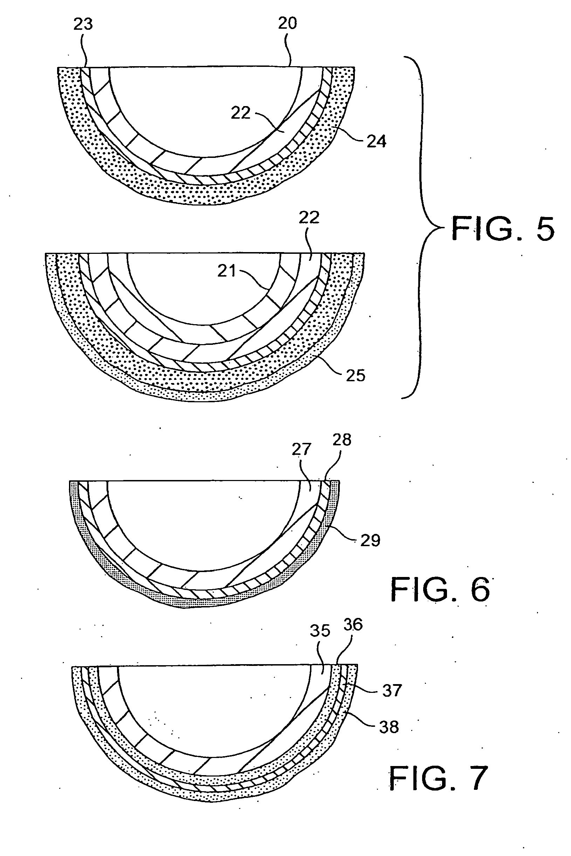

Figure 5 shows a method and construction which utilises a preformed metal shell which can be used as an insert. The shell is indicated by reference numeral 20 and has an inner surface of specified structure, roughness, and retentive features to permit engagement of a plastic composite bearing surface, indicated by reference numeral 21.

The metal preform may be made as a graded metal structure by, for example, laser sintering using titanium and having an overall thickness of 2 - 3 mm. The preform comprises an inner surface layer 22 which is porous to retain the plastic composite bearing surface 21, a dense layer 23 which acts as a barrier layer to stop ingress of the plastic/composite into the metallic structure and an outer layer 24 which is of controlled interconnected porosity and is intended for bone ingrowth. This has a nominal porosity of 400 µm which is able to sustain the bone ingrowth referred to above.

The preformed metal insert is made as shown at the upper part of Figure 5 and the composite material bearing surface layer 21 is subsequently moulded to it. The outer surface of the metallic structure is then coated with HAP, indicated by reference numeral 25, by sputtering or chemical deposition.

The particle size of the porous layer 22 can be 1 mm to allow the composite to infiltrate and to be retained.

In the construction shown in Figure 6 the bearing layer 27 is made in a similar manner to the arrangements shown in Figures 1 to 4, for example by injection moulding, and is then coated with a thin titanium layer 28 coating using plasma vapour deposition (PVD) in order to form a very thin titanium barrier between the composite material and the bone cells. A layer of hydroxyapatite 29 is then applied to form a continuous layer without damaging the titanium layer previously applied.

In the construction and method shown in Figure 7 a composite bearing surface layer 35 is formed by a similar process to that used in the previous Figures 1 to 4 by injection moulding. PEEK particles are sputtered by a plasma torch to provide a predetermined roughness in a layer indicated by reference numeral 36. A thin titanium layer 37 is now applied by a plasma torch under vacuum to form a barrier between the PEEK material layer 36 and the bone cells and the porous structure is then coated with a layer of hydroxyapatite, indicated by reference numeral 38, by any process that will provide a continuous layer.

In all the above examples the composite material is preferably PEEK reinforced with 30 % carbon fibres produced to actual shape by injecting moulding. The part can also be formed by a combination of moulding/extrusion and machining to final shape.

Any material such as tantalum or niobium could be used as an alternative to titanium.

The hydroxyapatite bioactive layer can be replaced by a coating with bone morphogenic proteins in any of the examples.

As described above the hydroxyapatite layer can be applied by plasma torch sputtering for non-porous surfaces. Onto porous surfaces, deposition process or any process that will ensure full covering of the open porous surface and allow thickness control can be applied. Such processes can be deposition or laser ablation.

## Claims

1. A method of forming a prosthetic acetabular cup which includes moulding a bearing liner (1) from a plastics material to form an inner bearing surface and attaching thereto a metallic backing layer (2) with a roughened or porous outer surface ***characterised in that*** said plastics material is PEEK resin and 20% to 40% carbon fibres, said metallic backing layer (2) is formed by plasma vapour deposition, sputtering metal particles (3) on the outer non-bearing surface of the bearing layer or forming said metallic backing layer (2) by laser sintering to provide a porous inner surface layer (22), a dense barrier layer (23) and an outer layer (24) with controlled inter-connected porosity intended for bone ingrowth; and coating the outer surface of said cup with bioactive materials (3) (25) by sputtering or chemical deposition.

2. A method as claimed in claim 1 in which the backing layer (2)(20) is made from titanium, tantalum or niobium.

3. A method as claimed in claim 1 or claim 2 in which the bioactive material (3) s hydroxyapatite (HAP) or bone morphogenic proteins (BMP).

4. A method as claimed in claims 1 to 3 in which the backing layer 2 has a porosity which varies from its inner to its outer sides to form an outer porous surface.

5. A method as claimed in claims 1 to 4 in which the metallic backing layer (2) formed by sputtering metal particles is formed by varying the sizes of the metal particles to form an inter connected porosity which increases towards the outer surface as the layer is built up.

6. A method as claimed in claim 5 in which the particle size increases from smaller to larger for the increasing porosity.

7. A method as claimed in any one of claims 1 to 3 in which the metallic backing layer (2) is formed by sputtering metal particles, and in which a further layer (10) is formed on the backing layer (2) by sputtering a mixture of metal and HAP particles thereon and before applying said coating (11) of bioactive materials.

8. A method as claimed in claims 1 to 3 in which the metallic backing layer (13) is formed by sputtering metal particles and which includes a titanium structure (14) obtained by a laser sintering process over said metallic backing layer and in which said bioactive material coating (15) is applied over said titanium structure.

9. A method as claimed in claims 1 to 3 in which said backing layer (20) is formed by laser sintering and has an overall thickness of 2-3 mm.

10. A method as claimed in claims 1 to 3 in which the metallic backing layer (36) is formed by sputtering metal particles and which includes applying PEEK particles by sputtering by a plasma torch to the bearing layer to provide a predetermined roughness and then applying a thin titanium layer (37) by plasma torch under vacuum prior to coating with the bioactive material.

11. A method as claimed in any one of the preceding claims which includes providing fixation features (16).

12. A method as claimed in claim 10 in which said fixation features (16) are fins or spikes.

## Patentansprüche

1. Verfahren zur Bildung einer prothetischen Hüftpfanne, umfassend das Formen einer Lagerschale (1) aus einem Kunststoffmaterial zur Bildung einer inneren Lagerfläche und Anbringen daran einer metallischen Stützschicht (2) mit einer angerauhten oder porösen Außenfläche, **dadurch gekennzeichnet, daß** das Kunststoffmaterial PEEK-Harz und 20 bis 40 % Kohlefasern ist, die metallische Stützschicht (2) durch Plasmaaufdampfung gebildet wird, wobei Metallteilchen (3) auf die äußere Nicht-Lagerfläche der Lagerschicht gesputtert werden oder die metallische Stützschicht (2) durch Lasersintern zur Bereitstellung einer porösen inneren Oberflächenschicht (22), einer dichten Sperrschicht (23) und einer Außenschicht (24) mit kontrollierter verbundener Porosität, vorgesehen für den Knocheneinwuchs, gebildet wird; und die Außenfläche der Pfanne mit bioaktiven Materialien (3) (25) durch Sputtern oder chemische Abscheidung beschichtet wird.

2. Verfahren nach Anspruch 1, wobei die Stützschicht (2) (20) aus Titan, Tantal oder Niob ist.

3. Verfahren nach Anspruch 1 oder Anspruch 2, wobei das bioaktive Material (3) Hydroxyapatit (HAP) oder morphogenetische Knochenproteine (BMP) ist.

4. Verfahren nach den Ansprüchen 1 bis 3, wobei die Stützschicht 2 eine Porosität aufweist, die von ihrer Innen- zu ihrer Außenseite variiert, wodurch eine poröse Außenfläche gebildet wird.

5. Verfahren nach den Ansprüchen 1 bis 4, wobei die durch Sputtern von Metallteilchen gebildete metallische Stützschicht (2) durch Variieren der Größen der Metallteilchen unter Bildung einer verbundenen Porosität, die beim Aufbau der Schicht in Richtung der Außenfläche zunimmt, gebildet wird.

6. Verfahren nach Anspruch 5, wobei sich die Teilchengröße für eine zunehmende Porosität von kleiner zu größer erhöht.

7. Verfahren nach einem der Ansprüche 1 bis 3, wobei die metallische Stützschicht (2) durch Sputtern von Metallteilchen gebildet wird, und wobei eine weitere Schicht (10) auf der Stützschicht (2) durch Sputtern eines Gemisches aus Metall- und HAP-Teilchen darauf und vor der Auftragung der Beschichtung (11) aus bioaktiven Materialien gebildet wird.

8. Verfahren nach den Ansprüchen 1 bis 3, wobei die metallische Stützschicht (13) durch Sputtern von Metallteilchen gebildet wird und eine Titanstruktur (14), erhalten durch ein Lasersinterverfahren, über der metallischen Stützschicht umfaßt, und wobei die Beschichtung (15) aus bioaktivem Material über der Titanstruktur aufgetragen wird.

9. Verfahren nach den Ansprüchen 1 bis 3, wobei die Stützschicht (20) durch Lasersintern gebildet wird und eine Gesamtdicke von 2 bis 3 mm hat.

10. Verfahren nach den Ansprüchen 1 bis 3, wobei die metallische Stützschicht (36) durch Sputtern von Metallteilchen gebildet wird, und das das Auftragen von PEEK-Teilchen durch Sputtern mittels eines Plasmabrenners auf die Lagerschicht, um so eine vorbestimmte Rauheit zu liefern, und dann das Auftragen einer dünnen Titanschicht (37) mittels eines Plasmabrenners unter Vakuum, vor dem Beschichten mit dem bioaktiven Material, umfaßt.

11. Verfahren nach einem der vorhergehenden Ansprüche, das die Bereitstellung von Fixierelementen (16) umfaßt.

12. Verfahren nach Anspruch 10, wobei die Fixierelemente (16) Rippen oder Zacken sind.

## Revendications

1. Procédé de formation d'un cotyle prothétique qui comprend le moulage d'un revêtement de support (1) à partir d'un matériau plastique pour former une surface de support interne et la fixation à celui-ci d'une couche de renfort métallique (2) avec une surface externe rugueuse ou poreuse, **caractérisé en ce que** ledit matériau plastique est constitué d'une résine PEEK et de 20 % à 40 % de fibres de carbone, ladite couche de renfort métallique (2) est formée par dépôt en phase vapeur activé par plasma, on pulvérise des particules métalliques (3) sur la surface non-porteuse externe de la couche de support ou on forme ladite couche de renfort métallique (2) par frittage laser pour fournir une couche de surface interne poreuse (22), une couche de barrière dense (23) et une couche externe (24) avec une porosité interconnectée contrôlée prévue pour l'interposition osseuse ; et on applique sur la surface externe dudit cotyle des matériaux bioactifs (3) (25) par pulvérisation ou dépôt chimique.

2. Procédé selon la revendication 1, dans lequel la couche de renfort (2) (20) est constituée de titane, tantale ou niobium.

3. Procédé selon la revendication 1 ou la revendication 2, dans lequel le matériau bioactif (3) est de l'hydroxyapatite (HAP) ou des protéines morphogénétiques osseuses (BMP).

4. Procédé selon les revendications 1 à 3, dans lequel la couche de renfort (2) a une porosité qui varie de son côté interne à son côté externe pour former une surface poreuse externe.

5. Procédé selon les revendications 1 à 4, dans lequel la couche de renfort métallique (2) formée par pulvérisation de particules métalliques est formée en modifiant les tailles des particules métalliques pour former une porosité interconnectée qui augmente vers la surface externe au fur et à mesure de la constitution de la couche.

6. Procédé selon la revendication 5, dans lequel la taille des particules augmente de plus petite à plus grande pour l'augmentation de la porosité.

7. Procédé selon l'une quelconque des revendications 1 à 3, dans lequel la couche de renfort métallique (2) est formée par pulvérisation de particules métalliques, et dans lequel une autre couche (10) est formée sur la couche de renfort (2) par pulvérisation d'un mélange de particules métalliques et HAP sur celle-ci et avant application dudit revêtement (11) de matériaux bioactifs.

8. Procédé selon les revendications 1 à 3, dans lequel la couche de renfort métallique (13) est formée par pulvérisation de particules métalliques et comprend une structure de titane (14) obtenue par un procédé de frittage laser sur ladite couche de renfort métallique et dans lequel ledit revêtement de matériau bioactif (15) est appliqué sur ladite structure de titane.

9. Procédé selon les revendications 1 à 3, dans lequel ladite couche de renfort (20) est formée par frittage laser et a une épaisseur globale de 2 à 3 mm.

10. Procédé selon les revendications 1 à 3, dans lequel la couche de renfort métallique (36) est formée par pulvérisation de particules métalliques et comprend l'application de particules PEEK par pulvérisation au moyen d'un chalumeau à plasma sur la couche de support pour fournir une rugosité prédéterminée, et ensuite, l'application d'une fine couche de titane (37) au moyen d'un chalumeau à plasma sous vide avant revêtement avec le matériau bioactif.

11. Procédé selon l'une quelconque des revendications précédentes, qui comprend de prévoir des moyens de fixation (16).

12. Procédé selon la revendication 10, dans lequel lesdits moyens de fixation (16) sont des ailettes ou des pointes.
